(19) Europäisches Patentamt / European Patent Office / Office européen des brevets

(11) **EP 4 691 468 A1**

(12) **EUROPEAN PATENT APPLICATION**
published in accordance with Art. 153(4) EPC

(43) Date of publication:
**11.02.2026 Bulletin 2026/07**

(21) Application number: **24784977.1**

(22) Date of filing: **05.04.2024**

(51) International Patent Classification (IPC):
***A61K 31/513*** *(2006.01)* ***A61K 9/107*** *(2006.01)*
***A61K 31/635*** *(2006.01)* ***A61K 47/54*** *(2017.01)*
***A61P 1/04*** *(2006.01)* ***A61P 43/00*** *(2006.01)*

(52) Cooperative Patent Classification (CPC):
**A61K 9/107; A61K 31/513; A61K 31/635; A61K 47/54; A61P 1/04; A61P 43/00**

(86) International application number:
**PCT/JP2024/014041**

(87) International publication number:
**WO 2024/210195 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.04.2023 JP 2023061838**
**12.05.2023 JP 2023079352**

(71) Applicant: **EA Pharma Co., Ltd.**
**Tokyo 104-0042 (JP)**

(72) Inventors:
- **TAGATA Yusuke**
  **Tokyo 104-0042 (JP)**
- **SEKI Tatsunori**
  **Tokyo 104-0042 (JP)**
- **TSURUTA Atsushi**
  **Tokyo 104-0042 (JP)**

(74) Representative: **Mewburn Ellis LLP**
**Aurora Building**
**Counterslip**
**Bristol BS1 6BX (GB)**

# (54) PHARMACEUTICAL COMPOSITION FOR TREATING INFLAMMATORY BOWEL DISEASE

(57) To provide a pharmaceutical composition having a dosage of a compound represented by formula (I) or a pharmaceutically acceptable salt thereof is effective in the treatment of inflammatory bowel disease. Disclosed is a pharmaceutical composition for treating inflammatory bowel disease, which comprises a compound represented by formula (I) or a pharmaceutically acceptable salt thereof, wherein the compound or a pharmaceutically acceptable salt thereof is administered to a human in an amount of 50 mg or more as an active ingredient per day. (R represents a hydroxy group or an isobutyloxy group.)

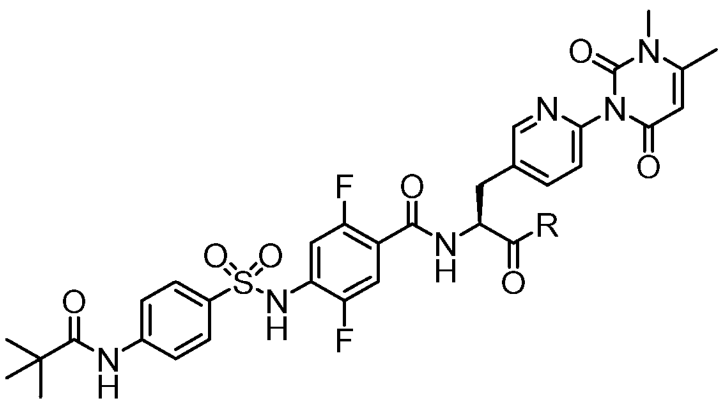

(I)



Processed by Luminess, 75001 PARIS (FR)

(Cont. next page)

FIG.4

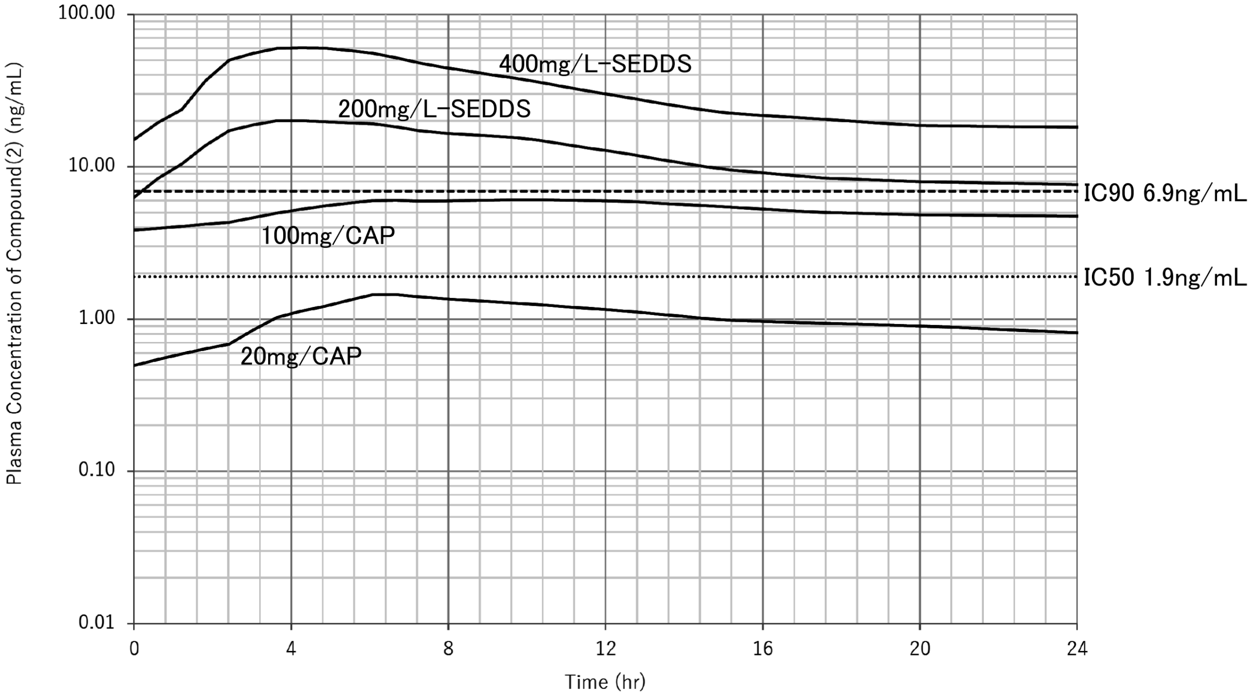
100.00
10.00
1.00
0.10
0.01
Plasma Concentration of Compound(2) (ng/mL)
0
4
8
12
16
20
24
Time (hr)
400mg/L-SEDDS
200mg/L-SEDDS
100mg/CAP
20mg/CAP
IC90 6.9ng/mL
IC50 1.9ng/mL

## Description

Technical Field

The present disclosure relates to a pharmaceutical composition useful as a therapeutic agent for inflammatory bowel disease.

Background Art

Inflammatory bowel disease (IBD) refers to diseases that cause inflammation in the intestines, primarily represented by ulcerative colitis (UC) and Crohn's disease (CD). The cause is unknown, and there is still no cure, but the basic treatment philosophy is to administer remission induction therapy during the active phase and continue remission maintenance therapy after remission induction (Riichi Hisamatsu, Ulcerative Colitis and Crohn's Disease Diagnostic Criteria and Treatment Guidelines (Revised Edition for Fiscal Year 2021)).

Conventional treatment has centered on 5-aminosalicylic acid preparations (sometimes referred to as 5-ASA preparations) and corticosteroids, but in recent years, anti-$\alpha 4\beta 7$ integrin antibody preparations have been used.

On the other hand, the development of orally administrable low-molecular-weight compounds has also progressed, and compounds of the following formula (I) have been reported (International Publication No. WO2017/135472).

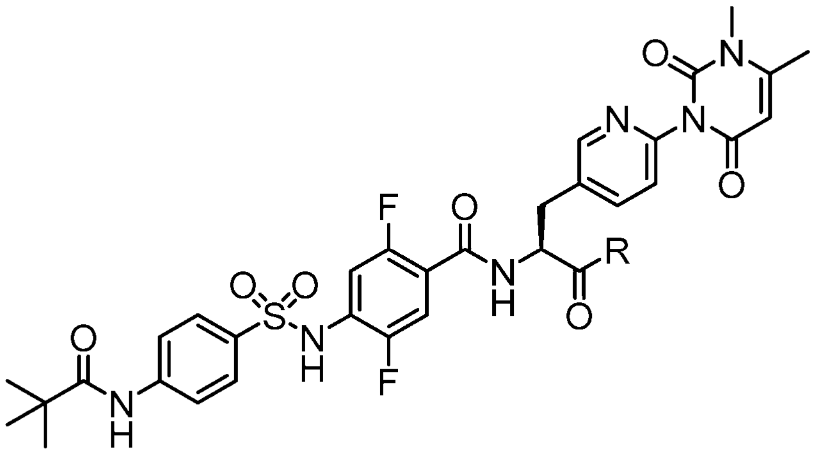

(I)

In formula (I), R represents a hydroxy group or an isobutyloxy group.

When R is an isobutyloxy group, it is the ester shown in the following formula (1) (sometimes referred to as "Compound (1)" in the present specification), and when R is a hydroxy group, it is the carboxylic acid shown in the following formula (2) (sometimes referred to as "Compound (2)" in the present specification). The ester of formula (1) is presumed to be absorbed as a prodrug and converted to the active metabolite of formula (2) in vivo.

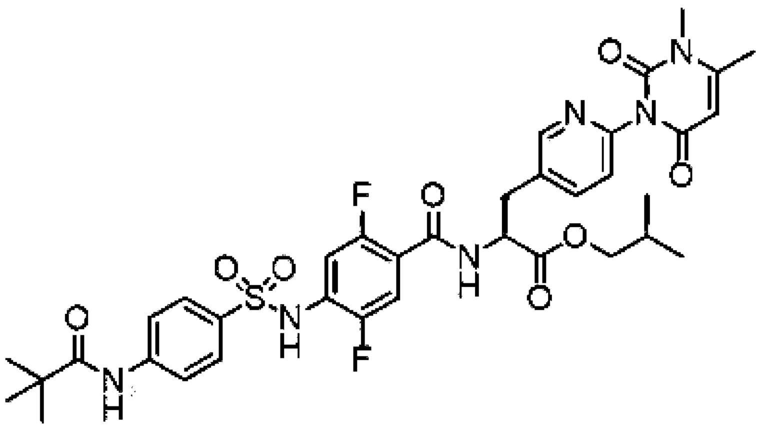

(1)

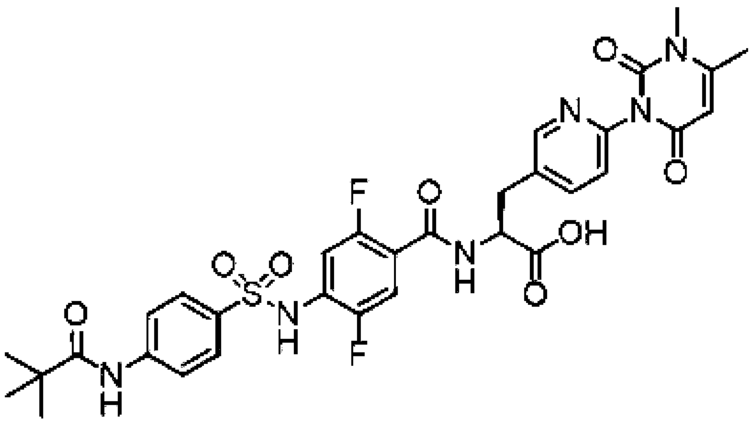

(2)

Summary of Invention

As described above, various therapeutic agents for inflammatory bowel disease have been developed, but 5-ASA preparations and corticosteroids may be insufficiently effective, and antibody preparations have drawbacks such as complex pharmacokinetics, limited administration methods, high production costs, and secondary inefficacy due to the induction of anti-drug antibodies attributable to high-molecular-weight proteins. On the other hand, low-molecular-weight compounds can overcome the drawbacks of antibody preparations, but even with low-molecular-weight compounds, it has been difficult to predict the effective dosage in the treatment of inflammatory bowel disease solely from the chemical structure and the like, due to interactions with a wide variety of substances in vivo and complex pharmacokinetics.

As a result of diligent studies to solve the above problems, the inventors have found that a compound represented by formula (I) or a pharmaceutically acceptable salt thereof effectively acts on inflammatory bowel disease at a certain dosage.

That is, the present disclosure may have the following configurations:

[1] A pharmaceutical composition for the treatment of inflammatory bowel disease, comprising a compound represented by the following formula (I) or a pharmaceutically acceptable salt thereof, wherein the compound or a pharmaceutically acceptable salt thereof is administered to a human at a dose of 50 mg or more per day as an active ingredient,

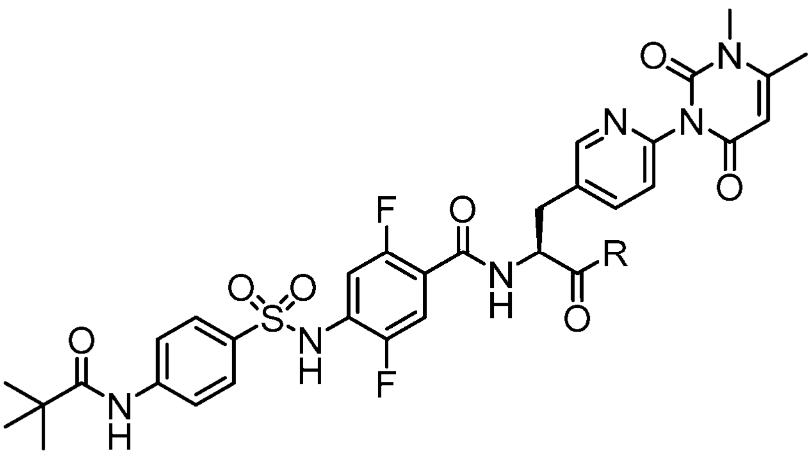

(I)

where R represents a hydroxy group or an isobutyloxy group.

[2] The pharmaceutical composition according to [1], wherein the compound or a pharmaceutically acceptable salt thereof is administered to a human at a dose of 100 mg or more per day as an active ingredient.

[3] The pharmaceutical composition according to [1], wherein the compound or a pharmaceutically acceptable salt thereof is administered to a human at a dose of 200 mg or more per day as an active ingredient.

[4] The pharmaceutical composition according to any one of [1] to [3], which is an oral dosage form.

[5] The pharmaceutical composition according to any one of [1] to [4], wherein the compound or a pharmaceutically acceptable salt thereof is repeatedly administered for 2 days or more.

[6] The pharmaceutical composition according to any one of [1] to [5], wherein the compound or a pharmaceutically acceptable salt thereof is administered 1 to 5 times a day.

[7] The pharmaceutical composition according to any one of [1] to [6], which is in a form of a self-emulsifying preparation.

[8] The pharmaceutical composition according to any one of [1] to [7], wherein the compound or a pharmaceutically acceptable salt thereof is repeatedly administered for 2 days or more so that a plasma concentration as a trough value of the compound represented by the following formula (2) is 1.9 ng/mL or more.

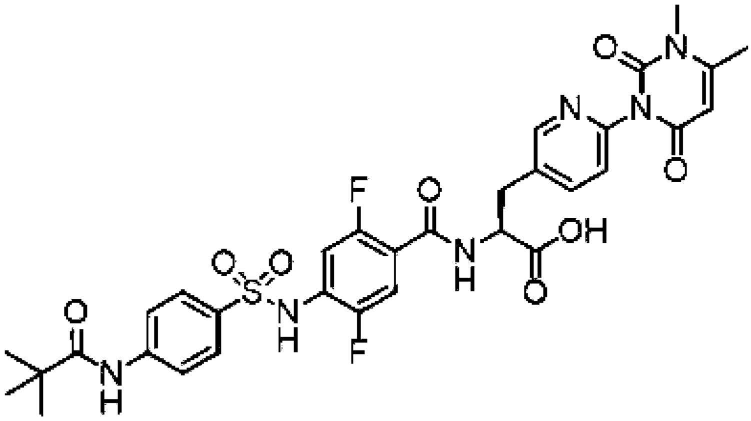

(2)

[9] The pharmaceutical composition according to any one of [1] to [7], wherein the compound or a pharmaceutically acceptable salt thereof is repeatedly administered for 2 days or more so that a plasma concentration as a trough value of the compound represented by the above formula (2) is 6.9 ng/mL or more.

[10] The pharmaceutical composition according to any one of [1] to [9], wherein the inflammatory bowel disease is ulcerative colitis.

[11] The pharmaceutical composition according to any one of [1] to [10], wherein the compound or a pharmaceutically acceptable salt thereof is administered to a human at a dose of 800 mg or less per day as an active ingredient.

[A] A method of treating inflammatory bowel disease, comprising administering a therapeutically effective amount of a compound represented by formula (I) as defined in [1] above or a pharmaceutically acceptable salt thereof to a patient in need of treatment.

[B] The compound represented by formula (I) as defined in [1] above or a pharmaceutically acceptable salt thereof for use in the treatment of inflammatory bowel disease.

[C] Use of the compound represented by formula (I) as defined in [1] above or a pharmaceutically acceptable salt thereof in the treatment of inflammatory bowel disease.

[D] Use of the compound represented by formula (I) as defined in [1] above or a pharmaceutically acceptable salt thereof in the production of a medicament for the treatment of inflammatory bowel disease.

By using the pharmaceutical composition of the present disclosure, inflammatory bowel disease can be effectively treated.

Brief Description of Drawings

Fig. 1A is a diagram showing the plasma concentration profile of Compound (2) after single-dose administration.

Fig. 1B is a diagram showing the plasma concentration profile of Compound (2) after single-dose administration.

Fig. 2A is a diagram showing the 24-hour post-dose trough value profile of Compound (2) after repeated administration.

Fig. 2B is a diagram showing the 12-hour post-dose trough value profile of Compound (2) after repeated administration.

Fig. 3A is a diagram showing the plasma concentration profile of Compound (2) after repeated administration (L-SEDDS 400 mg once daily) on day 10 and after single-dose administration.

Fig. 3B is a diagram showing the plasma concentration profile of Compound (2) after repeated administration (C-SEDDS 200 mg twice daily) on day 10 and after single-dose administration.

Fig. 4 is a diagram showing the predicted plasma concentration profile of Compound (2) on day 10 of repeated administration.

Fig. 5 is a diagram showing the concentration of Compound (2) in collected plasma and ligand binding inhibitory activity.

Fig. 6 is a diagram showing the predicted plasma concentration profile of Compound (2) on day 10 of repeated administration twice daily.

Description of Embodiments

The pharmaceutical composition of the present disclosure contains, as an active ingredient, a compound represented by the following formula (I) (hereinafter sometimes simply referred to as Compound (I)) or a pharmaceutically acceptable salt thereof. Compound (I) or a pharmaceutically acceptable salt thereof can act extremely effectively on patients with inflammatory bowel disease.

Compound (I) can be produced, for example, by the method described in International Publication No. WO2017/135472 (Compound A45, Compound P97).

In the present disclosure, the term "pharmaceutically acceptable salt" is not particularly limited as long as Compound (I) can form a salt while retaining pharmacological activity. For example, for acidic groups such as the carboxyl group in formula (I), examples include ammonium salts; alkali metal salts such as sodium and potassium; alkaline earth metal salts such as calcium and magnesium; aluminum salts; zinc salts; salts with organic amines such as triethylamine, ethanolamine, morpholine, piperidine, and dicyclohexylamine; and salts with basic amino acids such as arginine and lysine. For basic groups in formula (I), examples include salts with inorganic acids such as hydrochloric acid, sulfuric acid, and phosphoric acid; salts with organic carboxylic acids such as acetic acid, citric acid, benzoic acid, maleic acid, fumaric acid, tartaric acid, and succinic acid; and salts with organic sulfonic acids such as methanesulfonic acid and p-toluenesulfonic acid. These also apply to pharmaceutically acceptable salts of Compound (1) and Compound (2).

As a method for forming the above salts, known methods can be appropriately used. For example, a method of mixing Compound (I) and a necessary acid or base in a suitable ratio in a solvent or dispersant, or a method of subjecting Compound (I) in the form of another salt to cation exchange or anion exchange can be mentioned.

Further, Compound (I) also includes its solvates, such as hydrates and alcohol adducts.

The pharmaceutical composition of the present disclosure is useful for the treatment of inflammatory bowel disease. "Treatment" in the present specification includes preventing recurrence of inflammatory bowel disease, suppressing the progression of inflammatory bowel disease, alleviating the symptoms of inflammatory bowel disease, maintaining remission of inflammatory bowel disease, and curing inflammatory bowel disease.

Examples of inflammatory bowel disease include ulcerative colitis and Crohn's disease.

The inflammatory bowel disease for which the pharmaceutical composition of the present disclosure is particularly useful is, for example, inflammatory bowel disease in which an $\alpha 4$ integrin-dependent adhesion process is involved in the pathology.

Further, the inflammatory bowel disease for which the pharmaceutical composition of the present disclosure is particularly useful is, for example, ulcerative colitis.

<Dose Per Day>

Compound (I) or a pharmaceutically acceptable salt thereof is administered to the target human at a dose of 50 mg or more per day as an active ingredient, preferably at a dose of 100 mg or more per day as an active ingredient, more preferably at a dose of 200 mg or more per day, further preferably at a dose of 300 mg or more per day, and even more preferably at a dose of 400 mg or more per day. The upper limit is not particularly limited, but is preferably 1000 mg or less per day, and more preferably 800 mg or less per day.

Among these, Compound (I) or a pharmaceutically acceptable salt thereof is particularly preferably administered to the target human at a dose of 100 to 800 mg, 200 to 800 mg, 300 to 800 mg, or 400 to 800 mg per day, and most preferably at a dose of 400 mg per day.

<Number of Doses Per Day>

There is no particular restriction on the number of doses per day. For example, Compound (I) or a pharmaceutically acceptable salt thereof can be administered to the target human 1 to 5 times a day. Among these, it is preferably administered 1 to 4 times a day, more preferably 1 to 3 times a day, and most preferably once or twice a day.

Further, when administering multiple times a day, the dose per administration is preferably an amount obtained by dividing the above-mentioned dose per day by the number of doses per day.

Among these, Compound (1) or a pharmaceutically acceptable salt thereof is preferably administered to the

target human once or twice a day at a dose of 100 mg or more per administration, more preferably once or twice a day at a dose of 100 to 800 mg per administration, even more preferably once or twice a day at a dose of 200 to 400 mg per administration, and most preferably once a day at a dose of 400 mg per administration or twice a day at a dose of 200 mg per administration.

As another embodiment, Compound (I) or a pharmaceutically acceptable salt thereof is preferably administered to the target human twice a day at a dose of 50 mg or more per administration, more preferably twice a day at a dose of 50 to 400 mg per administration, and even more preferably twice a day at a dose of 100 to 200 mg per administration.

<Administration Period>

The administration period is not particularly limited, and for example, it can be administered until the symptoms of inflammatory bowel disease in the target human improve. Compound (1) or a pharmaceutically acceptable salt thereof is preferably continuously administered for 2 days or more, more preferably continuously administered for 7 days or more, and even more preferably continuously administered for 10 days or more. Among these, it is preferable to administer Compound (I) or a pharmaceutically acceptable salt thereof for at least 4 weeks, more preferably for at least 8 weeks, and even more preferably for at least 1 year.

<Administration Target>

In the present disclosure, the preferred target "human" is a patient with inflammatory bowel disease, and the more preferred target is a patient with ulcerative colitis.

<Dosage Form>

The pharmaceutical composition of the present disclosure may be in the form of an oral preparation, intravenous injection, enema preparation, suppository, etc., but it is preferably in the form of an oral preparation. Examples of oral preparations include tablets, powders, pills, granules, capsules, solutions, sugar-coated tablets, depot preparations, and syrups, each of which can be produced according to conventional methods using general pharmaceutical excipients.

For example, tablets can be obtained by mixing Compound (I) or a pharmaceutically acceptable salt thereof as an active ingredient with known excipients, for example, inert diluents such as lactose, calcium carbonate, or calcium phosphate; binders such as gum arabic, cornstarch, or gelatin; disintegrants such as alginic acid, cornstarch, dextrin, pullulan, cornstarch, potato starch, or pregelatinized starch; sweeteners such as sucrose, lactose, or saccharin; flavoring agents such as peppermint, wintergreen oil, or cherry; glidants such as magnesium stearate, talc, magnesium oxide, magnesium lauryl sulfate, magnesium aluminometasilicate, or carboxymethylcellulose; surfactants such as sodium lauryl sulfate, polysorbate 80, sucrose fatty acid esters, polyoxyl 40 stearate, polyoxyl 35 castor oil, polyoxyethylene hydrogenated castor oil 60, sorbitan monostearate, sorbitan monopalmitate, and PEG-8 glyceryl caprylate; excipients such as sucrose, glucose, reduced maltose, mannitol, sorbitol, xylitol, and trehalose; excipients for soft gelatin capsules and suppositories such as fats, waxes, semi-solid and liquid polyols, natural oils, and hydrogenated oils; and excipients for solutions such as water, alcohol, glycerol, polyols, sucrose, invert sugar, glucose, and vegetable oils.

For example, examples of capsule preparations include those in which the pharmaceutical composition of the present disclosure is enclosed in hard capsules, soft capsules, etc. Hard capsule preparations are those in which a powder or granular pharmaceutical composition is filled into a capsule produced using gelatin or the like as a base, and soft capsules are those in which a base obtained by adding glycerin or sorbitol to gelatin is used to mainly wrap a liquid pharmaceutical composition and mold it into a capsule shape. There are various shapes such as spherical and oval shapes, and various sizes.

Furthermore, the pharmaceutical composition of the present disclosure may be in the form of a self-emulsifying preparation such as SEDDS, SMEDDS, and SNEDDS. Examples of such self-emulsifying preparations include, but are not limited to, liquid self-emulsifying drug delivery systems (L-SEDDS) and soft capsule self-emulsifying drug delivery systems (C-SEDDS).

The self-emulsifying preparation is not particularly limited as long as it can dissolve or disperse Compound (I) or a pharmaceutically acceptable salt thereof, and various surfactants and the like are used. Examples of these surfactants include lipophilic surfactants (e.g., polyglyceryl-2 dioleate, polyglyceryl-3 dioleate, polyglyceryl-10 trioleate, propylene glycol monolaurate, propylene glycol monocaprylate, glyceryl monocaprylate, glyceryl monolaurate, glyceryl dilaurate, glyceryl monolinoleate, etc.) and hydrophilic surfactants (e.g., polyoxyl 40 castor oil, polyoxyl 35 castor oil, PEG-25 trioleate, PEG-60 corn glycerides, PEG-60 almond oil, PEG-40 palm kernel oil, PEG-50 castor oil, PEG-50 hydrogenated castor oil, PEG-60 hydrogenated castor oil, PEG-8 glyceryl caprylate/caprate, lauroyl polyoxyl-32 glycerides, linoleoyl polyoxyl-6 glycerides, oleoyl polyoxyl-6 glycerides, stearoyl polyoxyl-32 glycerides, PEG-6 glyceryl caprylate/caprate, polyglyceryl-10 monooleate, polyglyceryl-10 laurate, polyglyceryl-15 hydroxystearate, $\alpha$-tocopheryl PEG 400 succinate,

α-tocopheryl PEG 1000 succinate, dl-α-tocopheryl PEG 1000 succinate, d-α-tocopheryl PEG 1000 succinate, etc.). In addition to the above surfactants, a co-solvent (e.g., acetic acid, sorbitol, mannitol, glycerol, triacetin, triethyl citrate, N-methylpyrrolidone, N-hydroxyethylpyrrolidone, polyvinylpyrrolidone, propylene carbonate, ethanol, propylene glycol, etc.) may be added.

These surfactants can be used alone or in combination.

<Plasma Concentration>

The pharmaceutical composition of the present disclosure is administered to humans at a dose that brings about a therapeutically effective improvement in inflammatory bowel disease. The effect depends on the maximum plasma concentration (Cmax) of Compound (2) and the steady-state plasma concentration (trough value) 6 hours, 8 hours, 12 hours, or 24 hours later.

For example, the pharmaceutical composition of the present disclosure is preferably administered so that the Cmax of Compound (2) is, after single-dose or repeated administration, 1.9 ng/mL or more, 2.0 ng/mL or more, 2.5 ng/mL or more, 3.0 ng/mL or more, 3.5 ng/mL or more, 4.0 ng/mL or more, 4.5 ng/mL or more, 5.0 ng/mL or more, 5.5 ng/mL or more, 6.0 ng/mL or more, 6.5 ng/mL or more, 6.6 ng/mL or more, 6.7 ng/mL or more, 6.8 ng/mL or more, 6.9 ng/mL or more, 7.0 ng/mL or more, 7.5 ng/mL, 8.0 ng/mL or more, 8.5 ng/mL or more, 9.0 ng/mL or more, 9.5 ng/mL or more, 10.0 ng/mL or more, 10.5 ng/mL or more, 11.0 ng/mL or more, 11.5 ng/mL or more, 12.0 ng/mL or more, 12.5 ng/mL or more, 13.0 ng/mL or more, 13.5 ng/mL or more, 14.0 ng/mL or more, 14.5 ng/mL or more, 15.0 ng/mL or more, 15.5 ng/mL or more, 16.0 ng/mL or more, 16.5 ng/mL or more, 17.0 ng/mL or more, 17.5 ng/mL or more, 18.0 ng/mL or more, 18.5 ng/mL or more, 19.0 ng/mL or more, 19.5 ng/mL or more, or 20.0 ng/mL or more, and more preferably administered so that it is 6.5 ng/mL or more, 6.6 ng/mL or more, 6.7 ng/mL or more, 6.8 ng/mL or more, 6.9 ng/mL or more, 7.0 ng/mL or more, 7.5 ng/mL, 8.0 ng/mL or more, 8.5 ng/mL or more, 9.0 ng/mL or more, 9.5 ng/mL or more, 10.0 ng/mL or more, 10.5 ng/mL or more, 11.0 ng/mL or more, 11.5 ng/mL or more, 12.0 ng/mL or more, 12.5 ng/mL or more, 13.0 ng/mL or more, 13.5 ng/mL or more, 14.0 ng/mL or more, 14.5 ng/mL or more, 15.0 ng/mL or more, 15.5 ng/mL or more, 16.0 ng/mL or more, 16.5 ng/mL or more, 17.0 ng/mL or more, 17.5 ng/mL or more, 18.0 ng/mL or more, 18.5 ng/mL or more, 19.0 ng/mL or more, 19.5 ng/mL or more, or 20.0 ng/mL or more.

For example, the pharmaceutical composition of the present disclosure is preferably administered so that the trough value of Compound (2) is 1.0 ng/mL or more, 1.5 ng/mL or more, 1.9 ng/mL or more, 2.0 ng/mL or more, 2.5 ng/mL or more, 3.0 ng/mL or more, 3.5 ng/mL or more, 4.0 ng/mL or more, 4.5 ng/mL or more, 5.0 ng/mL or more, 5.5 ng/mL or more, 6.0 ng/mL or more, 6.5 ng/mL or more, 6.6 ng/mL or more, 6.7 ng/mL or more, 6.8 ng/mL or more, 6.9 ng/mL or more, or 7.0 ng/mL or more, and more preferably administered so that it is 6.5 ng/mL or more, 6.6 ng/mL or more, 6.7 ng/mL or more, 6.8 ng/mL or more, 6.9 ng/mL or more, or 7.0 ng/mL or more.

The trough value refers to the plasma concentration of Compound (2) immediately before the next administration after administration of Compound (I) or a pharmaceutically acceptable salt thereof in multiple doses. Usually, the trough value refers to the minimum plasma concentration in the steady state, but for convenience, it is also referred to as the trough value in the present specification even when the steady state has not been reached. For example, when Compound (I) or a pharmaceutically acceptable salt thereof is repeatedly administered once daily for 2 days or more, the trough value refers to the plasma concentration 24 hours after the last administration in repeated administration, and this may be referred to as the 24-hour post-dose trough value. Further, when Compound (I) or a pharmaceutically acceptable salt thereof is administered twice daily for 1 day or 2 days or more, the trough value refers to the plasma concentration 12 hours after the last administration, and this may be referred to as the 12-hour post-dose trough value. When Compound (I) or a pharmaceutically acceptable salt thereof is administered three times daily for 1 day or 2 days or more, the trough value refers to the plasma concentration 8 hours after the last administration, and this may be referred to as the 8-hour post-dose trough value. When Compound (I) or a pharmaceutically acceptable salt thereof is administered four times daily for 1 day or 2 days or more, the trough value refers to the plasma concentration 6 hours after the last administration, and this may be referred to as the 6-hour post-dose trough value.

Furthermore, the pharmaceutical composition of the present disclosure can be increased or decreased in dosage based on the measured values of the trough concentration in plasma, and it is preferable that the trough value of the pharmaceutical composition in humans be adjusted to 1.9 ng/mL or more, and more preferably to 6.9 ng/mL or more. The pharmaceutical composition of the present disclosure is equipped with such a configuration, so that it is possible to safely and effectively treat this disease even when the systemic clearance varies greatly.

In another aspect, the present disclosure provides that the pharmacokinetic characteristics of the concentration-time curve, such as the time to reach Cmax (Tmax) and the area under the plasma concentration versus time curve (AUC), after single or multiple doses to human subjects are examined by statistical methods well established in the field of pharmacokinetics. The average value of pharmacokinetic parameters such as Cmax and AUC can be calculated by any of the methods of geometric mean, arithmetic mean, or median. In the present specification, unless otherwise specified, the average Cmax, average AUC, and average trough value are shown as arithmetic mean values. Even if the target average

value is calculated by a method different from that in the present specification, if the average value calculated according to the method of the present specification for the target falls within the numerical range described in the claims, it is intended that the target average value belongs to the numerical range described in the claims relating to the present disclosure.

<Concomitant Agent>

The pharmaceutical composition of the present disclosure can also be used in combination with other drugs having a therapeutic and/or preventive effect on inflammatory bowel disease.

Examples of other drugs having a therapeutic and/or preventive effect on inflammatory bowel disease include elemental diets (e.g., Elental (registered trademark) (Ajinomoto Co., Inc.)), 5-ASA preparations (e.g., mesalazine, Salazosulfapyridine (sulfasalazine)), corticosteroids (e.g., prednisolone, betamethasone, budesonide), and antibacterial agents (e.g., metronidazole).

Immunosuppressants (e.g., azathioprine, 6-mercaptopurine, cyclosporine, tacrolimus) can also be mentioned as drugs to be used in combination.

Examples of anti-cytokine drugs include anti-TNFα antibodies (e.g., infliximab, adalimumab, certolizumab pegol, golimumab), anti-IL-6 receptor antibodies (e.g., tocilizumab), anti-IL-12/23 antibodies (e.g., ustekinumab, briakinumab), anti-IL-17 receptor antibodies (e.g., AMG 827, AIN457), anti-α4β7 antibodies (vedolizumab), and low-molecular-weight drugs such as IL-12/23 production inhibitors (e.g., STA-5326), PDE-4 inhibitors (e.g., apremilast), chemokine inhibitors (e.g., vercimon, CCX507), Janus kinase inhibitors (e.g., tofacitinib, GLPG0634), S1P agonists (e.g., KRP-203, RPC1063), and α4 integrin inhibitors (e.g., carotegrast methyl).

Furthermore, it can also be used in combination with other treatment methods having a therapeutic and/or preventive effect on the above diseases, not limited to drugs. For example, leukocytapheresis (e.g., GCAP, LCAP) can be mentioned.

Therefore, the pharmaceutical composition of the present disclosure can be used in combination with other drugs having a therapeutic and/or preventive effect on the above diseases. Furthermore, the pharmaceutical composition of the present disclosure can be used in combination with other treatment methods for the above diseases.

When used in combination with other drugs, the pharmaceutical composition of the present disclosure and the other drugs may be administered simultaneously, consecutively, or separately with a time interval.

The dosage form of the drug to be used in combination is not particularly limited, such as oral agents, injections, enema preparations, and suppositories, but it is preferable to use enema preparations and suppositories in combination for patients with lesions near the anus.

Examples

Hereinafter, the present disclosure will be specifically described with reference to Examples. However, the present disclosure is not limited to these descriptions.

<Phase 1 Clinical Trial>

A phase 1 clinical trial was conducted to evaluate the safety, tolerability, and pharmacokinetics of Compound (1) when orally administered to healthy adult males.

[Clinical Trial Design]

This clinical trial was a single-center, randomized, double-blind, placebo-controlled, single and multiple ascending dose study with the primary objective of evaluating the safety and tolerability of Compound (1) when orally administered to healthy adult males.

Screening tests were conducted within 28 to 3 days before administration of the investigational drug to confirm the eligibility of the subjects, and the subjects were hospitalized 2 days before administration. After hospitalization, tests were conducted from 2 days before administration to the day of administration to confirm that there were no problems with eligibility, and then Compound (1) or placebo was randomly assigned. The subjects in each cohort consisted of 8 people, of which 6 were assigned to Compound (1) and 2 were assigned to placebo, and they were administered the drug.

In the single-dose administration study, Compound (1) or placebo was administered under fasting conditions.

In the repeated-dose administration study, Compound (1) or placebo was administered on day 1, and Compound (1) or placebo was repeatedly administered from day 8 to day 17. Day 8, when repeated administration was started, corresponds to administration day 1 (Day 1), and day 17 corresponds to administration day 10 (Day 10).

Hard capsules (CAP), a liquid self-emulsifying drug delivery system (L-SEDDS), and a soft capsule self-emulsifying drug delivery system (C-SEDDS) of Compound (1) and placebo were prepared as follows, repackaged

within the medical institution according to the dosage for each subject, and dispensed by pharmacy staff.

[Preparation of CAP]

Compound (1) at 1 mg, 10 mg, or 50 mg, or crystalline cellulose as placebo at 1 mg, 10 mg, or 50 mg was filled into hard capsules.

[Preparation of L-SEDDS]

Polyoxyl 35 castor oil (Kolliphor EL) and propylene glycol monocaprylate (Type II) (Capryol 90) were mixed at a mass ratio of about 7:1, this mixture was mixed with about 2% ethanol by mass ratio, Compound (1) was added to the resulting mixture so as to be about 10% by mass, and the mixture was sonicated at 40°C to produce a liquid self-emulsifying drug delivery system.

[Production of C-SEDDS]

Polyoxyl 35 castor oil (Kolliphor EL) and propylene glycol monocaprylate (Type II) (Capryol 90) were mixed at a mass ratio of about 7:1, this mixture was mixed with about 2% ethanol by mass ratio, Compound (1) was added to the resulting mixture so as to be about 10% by mass, and the self-emulsifying liquid obtained by stirring at 40°C was encapsulated in gelatin soft capsules in an amount of 1000 mg.

[Concentration Measurement (PK) of Compound (2)]

The plasma concentration of Compound (2) was measured using a validated measurement method. Specifically, plasma was separated from collected blood and processed, and then the plasma concentration was measured by an LC/MS/MS apparatus.

[MAdCAM-1 Binding Inhibition Rate (PD) of Lymphocytes in Plasma and $IC_{90}$, $IC_{50}$]

The MAdCAM-1 binding rate of lymphocytes in plasma was measured using a validated measurement method using the obtained blood samples, and the MAdCAM-1 binding inhibition rate of lymphocytes in plasma was calculated using Excel. Specifically, 5 μL of an 80 mM $MnCl_2$ solution was added to 90 μL of heparinized blood and incubated at room temperature for 10 minutes. A volume of 5 μL of 200 μg/mL recombinant human MAdCAM-1/Fc (R&D Systems) was added to make a total volume of 100 μL, and the mixture was incubated at room temperature for 30 minutes. Subsequently, 1900 μL of BD Phosflow Lyse/Fix Buffer 5X (BD Biosciences) was added, and hemolysis and fixation were performed at 37°C for 10 minutes. After centrifugation for 5 minutes, the supernatant was removed, and 2000 μL of RPMI-1640 medium supplemented with 10% inactivated fetal bovine serum (hereinafter referred to as medium) was added. After centrifugation for 5 minutes, the supernatant was removed and washed. After washing again with the medium, 50 μL of Brilliant Stain Buffer (BD Biosciences), 1.25 μL of MAdCAM-1 FITC (Hycult Biotech), 5 μL of CD4 PE (BD Biosciences), 2.5 μL of CD45RO APC (UCHL1) (BioLegend), 5 μL of CD3 BV421 (SK7) (BioLegend), and 2.5 μL of CD45 BV510 (HI30) (BioLegend) were added, and the mixture was incubated for 30 minutes. After washing with Stain Buffer (BSA) (BD Biosciences), the percentage of MAdCAM-1-positive cells among CD3-positive CD4-positive cells was measured using flow cytometry.

The percentage of MAdCAM-1-positive cells among CD3-positive CD4-positive cells was calculated by subtracting each measured value from the measured value when MAdCAM-1/Fc was not added as a control. The percentage of MAdCAM-1-positive cells among CD3-positive CD4-positive cells before administration of Compound (1) or placebo was set as 0% inhibition, and the MAdCAM-1 binding inhibition rate at each blood collection time point was obtained. Furthermore, the concentration $IC_{50}$ that causes 50% binding inhibition and the concentration $IC_{90}$ that causes 90% binding inhibition were calculated.

$$IC_{90}=6.9\ ng/mL$$

$$IC_{50}=1.9\ ng/mL$$

[Single-Dose Administration Study]

Compound (1) was orally administered once to 6 healthy adult males per dose and dosage form at doses of 6, 20, 100, 200, and 400 mg in the form of hard capsules (CAP), liquid self-emulsifying drug delivery system (L-SEDDS), or soft capsule self-emulsifying drug delivery system (C-SEDDS). After administration, blood was collected and Compound (2) was quantified. The average plasma concentrations of Compound (2) are shown in Table 1, and the plasma concentration profiles are shown in Figs. 1A and 1B.

Table 1: Average Plasma Concentrations (ng/mL) of Compound (2) after Single-Dose Administration

Table 1-1

| Time (h) | Dosage (mg)/Dosage Form | | | | |
|---|---|---|---|---|---|
| | 6/CAP | 20/CAP | 100/CAP | 200/L-SEDDS | 400/L-SEDDS |
| 0.5 | | | | 4.28 | 8.84 |
| 1 | 0.121 | 0.211 | 0.556 | 11.5 | 36.7 |
| 1.5 | 0.350 | 0.585 | 1.274 | 14.2 | 45.6 |
| 2 | 0.448 | 0.768 | 1.864 | 13.5 | 45.0 |
| 2.5 | 0.536 | 1.023 | 2.343 | 13.0 | 40.1 |
| 3 | 0.554 | 0.926 | 2.246 | 10.9 | 31.8 |
| 4 | 0.590 | 0.817 | 2.465 | 9.50 | 22.6 |
| 5 | 0.424 | 0.702 | 2.331 | 6.46 | 14.3 |
| 6 | 0.313 | 0.567 | 1.906 | 3.97 | 8.41 |
| 7 | 0.209 | 0.533 | 1.498 | 2.89 | 6.54 |
| 8 | 0.127 | 0.507 | 1.357 | 2.54 | 4.76 |
| 12 | 0.075 | 0.351 | 1.276 | 2.07 | 4.34 |
| 15 | | 0.338 | 1.135 | 2.00 | 3.90 |
| 21 | | 0.339 | 1.046 | 2.11 | 3.98 |
| 24 | | 0.245 | 1.030 | 2.25 | 3.79 |

Table 1-2

| Time (h) | Dosage (mg)/Dosage Form |
|---|---|
| | 200/C-SEDDS |
| 0.5 | Below quantitation limit |
| 1 | Below quantitation limit |
| 1.5 | 2.14 |
| 2 | 6.68 |
| 2.5 | 10.5 |
| 3 | 13.4 |
| 4 | 14.2 |
| 5 | 10.4 |
| 6 | 5.91 |
| 7 | 3.46 |
| 8 | 3.33 |
| 12 | 2.48 |
| 15 | 2.14 |

(continued)

| Time (h) | Dosage (mg)/Dosage Form |
|---|---|
| | 200/C-SEDDS |
| 21 | 1.85 |
| 24 | 1.69 |

[Repeated Administration Study 1]

Compound (1) was repeatedly administered to 6 healthy adult males at a dose of 400 mg once daily for 10 days as L-SEDDS, and blood was collected 24 hours later to quantify Compound (2).

The average plasma concentrations of Compound (2) are shown in Table 2, and the plasma concentration profile is shown in Fig. 2A.

Table 2: 24-Hour Post-Dose Trough Values (ng/mL) of Compound (2) after Repeated Administration

| Day | 400/L-SEDDS |
|---|---|
| Initial Value | 0.000 |
| 1 | 5.092 |
| 2 | 10.90 |
| 3 | 10.60 |
| 4 | 14.21 |
| 5 | 19.56 |
| 6 | 17.27 |
| 7 | 19.85 |
| 8 | 22.03 |
| 9 | 22.80 |
| 10 | 13.04 |

[Repeated Administration Study 2]

Compound (1) was repeatedly administered to 6 healthy adult males at a dose of 200 mg twice daily (every 12 hours) for 10 days as C-SEDDS, and blood was collected 12 hours later to quantify Compound (2).

The average plasma concentrations of Compound (2) are shown in Table 3, and the plasma concentration profile is shown in Fig. 2B.

Table 3: 12-Hour Post-Dose Trough Values (ng/mL) of Compound (2) after Repeated Administration

| Day | 400 (200×2)/C-SEDDS |
|---|---|
| Initial Value | 0.000 |
| 0.5 | 2.025 |
| 1 | 3.653 |
| 1.5 | 4.250 |
| 2 | 6.021 |
| 2.5 | 7.700 |
| 3 | 7.026 |
| 3.5 | 7.381 |
| 4 | 9.303 |
| 4.5 | 9.167 |

(continued)

| | |
|---|---|
| 5 | 9.985 |
| 5.5 | 12.59 |
| 6 | 11.20 |
| 6.5 | 10.81 |
| 7 | 11.82 |
| 7.5 | 10.83 |
| 8 | 14.16 |
| 8.5 | 12.12 |
| 9 | 15.06 |
| 9.5 | 12.20 |
| 10 | 13.69 |

As shown in Table 2 and Fig. 2A, with once-daily administration of L-SEDDS, the concentration was maintained above $IC_{90}$ (6.9 mg/mL) from the second day of administration. As shown in Table 3 and Fig. 2B, with twice-daily administration of C-SEDDS, the concentration was maintained above $IC_{90}$ (6.9 mg/mL) from the third day of administration.

Further, for once-daily administration of L-SEDDS and twice-daily administration of C-SEDDS, respectively, on the 10th day (Day 10) from the start of repeated administration, blood was collected at 0.5, 1, 1.5, 2, 2.5, 3, 4, 5, 6, 7, 8, 12, 15, 21, and 24 hours later to quantify Compound (2). For twice-daily administration of C-SEDDS, blood was further collected at 13, 14, 14.5, and 16 hours later to quantify Compound (2). The average plasma concentration profile of Compound (2) on the 10th day from the start of repeated administration is shown in Table 4, and the plasma concentration profiles are shown in Figs. 3A and 3B.

Table 4: Average Plasma Concentrations (ng/mL) of Compound (2) on Day 10 of Repeated Administration and after Single-Dose Administration

Table 4-1

| Time (h) | Single-dose administration | Day 10 of repeated administration |
|---|---|---|
| 0.5 | 8.84 | 23.82 |
| 1 | 36.7 | 43.52 |
| 1.5 | 45.6 | 44.71 |
| 2 | 45.0 | 40.35 |
| 2.5 | 40.1 | 34.19 |
| 3 | 31.8 | 32.09 |
| 4 | 22.6 | 28.31 |
| 5 | 14.3 | 25.14 |
| 6 | 8.41 | 17.77 |
| 7 | 6.54 | 14.77 |
| 8 | 4.76 | 13.57 |
| 12 | 4.34 | 16.86 |
| 15 | 3.90 | 11.33 |
| 21 | 3.98 | 15.69 |
| 24 | 3.79 | 13.04 |

Table 4-2

| Time (h) | Single-dose administration | Day 10 of repeated administration |
|---|---|---|
| 0.5 | Below quantitation limit | 11.82 |
| 1 | Below quantitation limit | 10.63 |
| 1.5 | 2.14 | 10.52 |
| 2 | 6.68 | 14.42 |
| 2.5 | 10.5 | 17.31 |
| 3 | 13.4 | 18.02 |
| 4 | 14.2 | 20.07 |
| 5 | 10.4 | 20.59 |
| 6 | 5.91 | 14.19 |
| 7 | 3.46 | 12.13 |
| 8 | 3.33 | 10.93 |
| 12 | 2.48 | 12.20 |
| 13 | | 15.64 |
| 14 | | 25.37 |
| 14.5 | | 25.16 |
| 15 | 2.14 | 22.99 |
| 16 | | 20.48 |
| 21 | 1.85 | 13.24 |
| 24 | 1.69 | 13.69 |

Table 4 (Table 4-1) and Fig. 3A show that the plasma concentration (trough value) of Compound (2) after 24 hours on the 10th day of repeated administration increased more than threefold. This suggests that repeated administration can maintain a high plasma concentration of Compound (2).

Further, Table 4 (Table 4-2) and Fig. 3B show that the plasma concentration (trough value) of Compound (2) 12 hours after the second dose on the 10th day of repeated administration increased about eightfold. This suggests that increasing the dose and repeated administration are useful for achieving a higher plasma concentration of Compound (2).

In addition, regarding the trough value of Compound (2), it was 2.25 ng/mL for a single dose of 200 mg once daily of L-SEDDS and 1.69 ng/mL for a single dose of 200 mg once daily of C-SEDDS. In single-dose administration, the trough value resulting from C-SEDDS was about 75% of the trough value resulting from L-SEDDS (Table 1), whereas the trough value on day 10 resulting from repeated administration of 200 mg twice daily of C-SEDDS (13.69 ng/mL) was equal to or higher than the trough value resulting from repeated administration of 400 mg once daily of L-SEDDS (13.04 ng/mL) (Tables 2-4). This suggests that even with the same daily dose, dividing the dose into twice daily maintains a higher plasma concentration. [PK Simulation Analysis 1 by Superposition Method]

The plasma concentration profile of Compound (2) on day 10 of repeated administration was predicted using the superposition method implemented in the Nonparametric Superposition (NPS) tool of the analysis software Phoenix WinNonlin, applying the single-dose results (Certara L.P.) (Fig. 4). The average plasma concentration profile at the following time points obtained from the simulation results is shown in Table 5.

Table 5: Predicted Plasma Concentration (ng/mL) of Compound (2) on Day 10 of Repeated Administration

| Time (h) | 20/CAP | 100/CAP | 200/L-SEDDS | 400/L-SEDDS |
|---|---|---|---|---|
| 0 | 0.50 | 3.84 | 6.29 | 15.05 |
| 0.6 | 0.54 | 3.94 | 8.37 | 19.48 |
| 1.2 | 0.59 | 4.07 | 10.42 | 23.73 |
| 1.8 | 0.64 | 4.19 | 13.78 | 36.61 |
| 2.4 | 0.69 | 4.32 | 17.24 | 50.08 |

(continued)

| Time (h) | 20/CAP | 100/CAP | 200/L-SEDDS | 400/L-SEDDS |
|---|---|---|---|---|
| 3 | 0.85 | 4.63 | 18.79 | 55.50 |
| 3.6 | 1.03 | 4.96 | 20.07 | 59.76 |
| 4.8 | 1.22 | 5.53 | 19.70 | 59.99 |
| 6.1 | 1.45 | 5.98 | 19.16 | 55.63 |
| 7.9 | 1.36 | 5.96 | 16.60 | 44.88 |
| 12.1 | 1.15 | 5.96 | 12.63 | 29.67 |
| 13.9 | 1.04 | 5.66 | 10.59 | 24.78 |
| 17 | 0.95 | 5.11 | 8.67 | 20.95 |
| 20 | 0.90 | 4.84 | 7.99 | 18.62 |
| 23 | 0.84 | 4.76 | 7.72 | 18.29 |
| 24 | 0.81 | 4.74 | 7.61 | 18.16 |

[EX vivo Integrin Inhibitory Activity]

The MAdCAM-1 binding inhibition rate (PD) of lymphocytes in plasma was evaluated using collected blood, and the correlation with the plasma concentration (PK) of Compound (2) was plotted (Fig. 5). Fig. 5 suggests that the plasma concentration and ligand inhibitory activity are correlated.

[PK Simulation Analysis 2 by Superposition Method]

The plasma concentration of Compound (2) on day 10 of repeated administration at 200 mg twice daily (every 12 hours) was predicted using the superposition method implemented in the Nonparametric Superposition (NPS) tool of the analysis software Phoenix WinNonlin, applying the results of once-daily administration at 200 mg (Certara L.P.) (Fig. 6). The average plasma concentration profile at the following time points obtained from the simulation results is shown in Table 6.

Table 6: Predicted Plasma Concentration (ng/mL) of Compound (2) on Day 10 of Repeated Administration Twice Daily

| Time (h) | 200 mg×2 times/day |
|---|---|
| 0 | 28.3 |
| 0.5 | 36.5 |
| 1 | 50.0 |
| 1.5 | 55.5 |
| 1.9 | 54.6 |
| 2.4 | 53.4 |
| 2.9 | 49.5 |
| 4.1 | 44.7 |
| 5.1 | 38.6 |
| 6.1 | 33.7 |
| 7 | 31.4 |
| 8 | 30.4 |
| 12 | 30.6 |
| 13.6 | 55.9 |
| 15 | 48.8 |

(continued)

| Time (h) | 200 mg×2 times/day |
|---|---|
| 21.1 | 30.0 |
| 24 | 28.8 |

Compared to the measured trough value (13.04 ng/mL) after repeated administration of 400 mg once daily, the predicted trough value (28.8 ng/mL) for repeated administration of 200 mg twice daily (trough value 12 hours after the second dose on day 10 of administration) maintained about twice the plasma concentration. This suggests that even with the same daily dose, dividing the dose into twice daily maintains a higher plasma concentration.

The measured and predicted Cmax and trough values are shown in Table 7. Since the effect of the drug depends on the plasma concentration, for each of Cmax and trough value, IC90 (6.9 ng/mL) or higher was judged as A, IC50 (1.9 ng/mL) or higher and less than IC90 (6.9 ng/mL) as B, and less than IC50 (1.9 ng/mL) as C.

Table 7

| | Administration Period | Administration Frequency | Dosage (mg)/ Dosage Form | Plasma Concentration (ng/mL) | | Judgment | |
|---|---|---|---|---|---|---|---|
| | | | | Cmax | Trough Value | Cmax | Trough Value |
| Measured Value | Single | Once | 6/CAP | 0.59 | - | C | C |
| | Single | Once | 20/CAP | 1.02 | 0.25 | C | C |
| | Single | Once | 100/CAP | 2.47 | 1.03 | B | C |
| | Single | Once | 200/L-SEDDS | 14.2 | 2.25 | A | B |
| | Single | Once | 200/C-SEDDS | 14.2 | 1.69 | A | C |
| | Single | Once | 400/L-SEDDS | 45.6 | 3.79 | A | B |
| | Repeated | Once | 400/L-SEDDS | 44.7 | 13.0 | A | A |
| | Repeated | Twice | 400(200×2)/ C-SEDDS | 25.4 | 13.7 | A | A |
| Predicted Value | Repeated | Once | 20/CAP | 1.45 | 0.81 | C | C |
| | Repeated | Once | 100/CAP | 6.09 | 4.74 | B | B |
| | Repeated | Once | 200/L-SEDDS | 20.1 | 7.61 | A | A |
| | Repeated | Once | 400/L-SEDDS | 60.3 | 18.2 | A | A |
| | Repeated | Twice | 400(200×2)/ L-SEDDS | 55.9 | 28.8 | A | A |

Furthermore, since the predicted Cmax on the 10th day of repeated administration at 20 mg was 1.45 ng/mL and the predicted 24-hour post-dose trough value was 0.81 ng/mL, it was estimated that the Cmax on the 10th day of repeated administration at 50 mg, which is 2.5 times the amount, would be 3.63 ng/mL and the trough value would be 2.03 ng/mL. These values were above IC50 (1.9 ng/mL) and could be judged as B.

## Claims

1. A pharmaceutical composition for the treatment of inflammatory bowel disease, comprising a compound represented by the following formula (I) or a pharmaceutically acceptable salt thereof, wherein the compound or pharmaceutically acceptable salt thereof is administered to a human at a dose of 50 mg or more per day as an active ingredient,

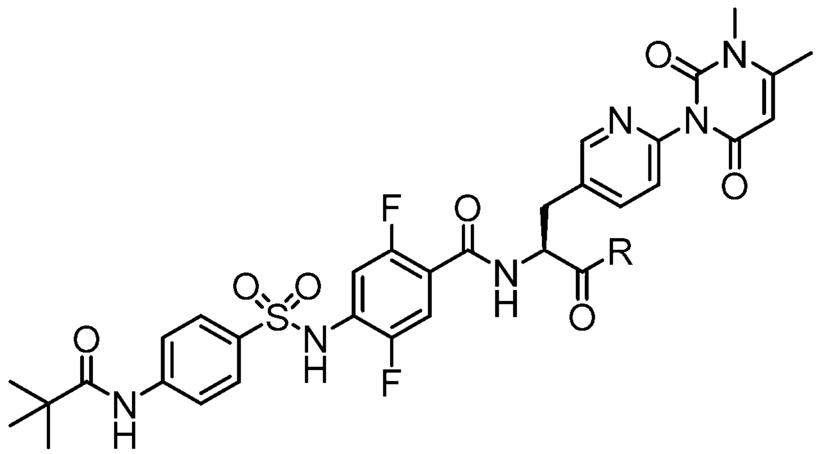

(I)

where R represents a hydroxy group or an isobutyloxy group.

**2.** The pharmaceutical composition according to claim 1, wherein the compound or pharmaceutically acceptable salt thereof is administered to a human at a dose of 100 mg or more per day as an active ingredient.

**3.** The pharmaceutical composition according to claim 1, wherein the compound or pharmaceutically acceptable salt thereof is administered to a human at a dose of 200 mg or more per day as an active ingredient.

**4.** The pharmaceutical composition according to any one of claims 1 to 3, which is an oral dosage form.

**5.** The pharmaceutical composition according to any one of claims 1 to 4, wherein the compound or pharmaceutically acceptable salt thereof is repeatedly administered for 2 days or more.

**6.** The pharmaceutical composition according to any one of claims 1 to 5, wherein the compound or pharmaceutically acceptable salt thereof is administered 1 to 5 times a day.

**7.** The pharmaceutical composition according to any one of claims 1 to 6, which is in a form of a self-emulsifying preparation.

**8.** The pharmaceutical composition according to any one of claims 1 to 7, wherein the compound or pharmaceutically acceptable salt thereof is repeatedly administered for 2 days or more so that a plasma concentration as a trough value of the compound represented by the following formula (2) is 1.9 ng/mL or more.

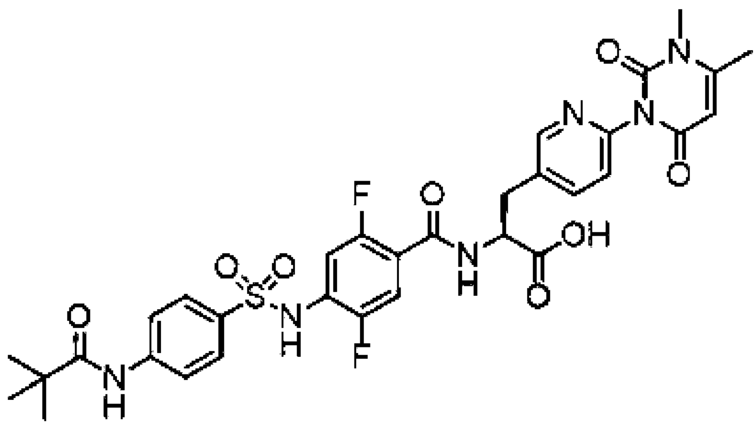

(2)

**9.** The pharmaceutical composition according to any one of claims 1 to 7, wherein the compound or pharmaceutically acceptable salt thereof is repeatedly administered for 2 days or more so that a plasma concentration as a trough value of the compound represented by the above formula (2) is 6.9 ng/mL or more.

**10.** The pharmaceutical composition according to any one of claims 1 to 9, wherein the inflammatory bowel disease is ulcerative colitis.

**11.** The pharmaceutical composition according to any one of claims 1 to 10, wherein the compound or pharmaceutically acceptable salt thereof is administered to a human at a dose of 800 mg or less per day as an active ingredient.

FIG.1A

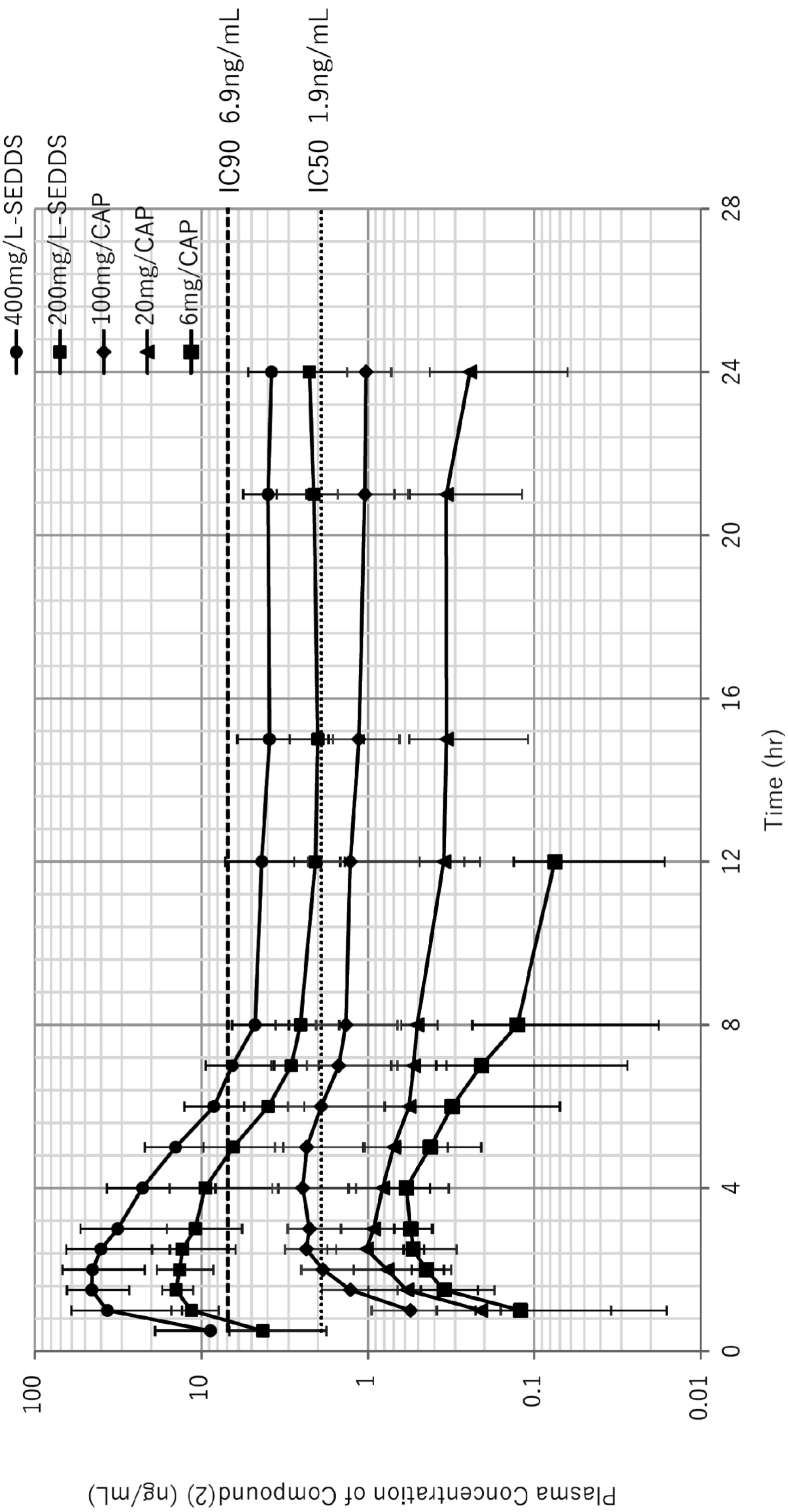
400mg/L-SEDDS
200mg/L-SEDDS
100mg/CAP
20mg/CAP
6mg/CAP
IC90 6.9ng/mL
IC50 1.9ng/mL
Plasma Concentration of Compound(2) (ng/mL)
100
10
1
0.1
0.01
Time (hr)
0
4
8
12
16
20
24
28

FIG.1B

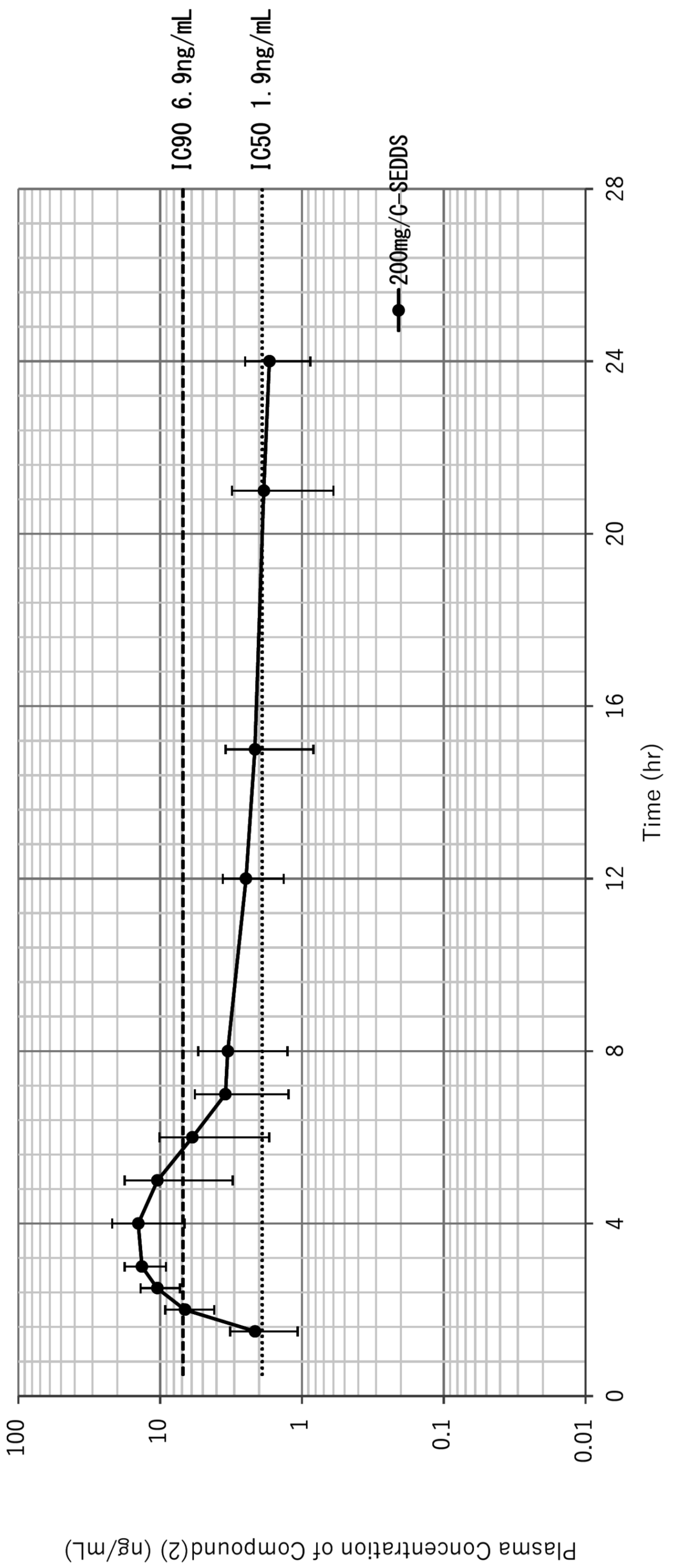
IC90 6.9ng/mL
IC50 1.9ng/mL
200mg/C-SEDDS
100
10
1
0.1
0.01
0
4
8
12
16
20
24
28
Time (hr)
Plasma Concentration of Compound(2) (ng/mL)

FIG.2A

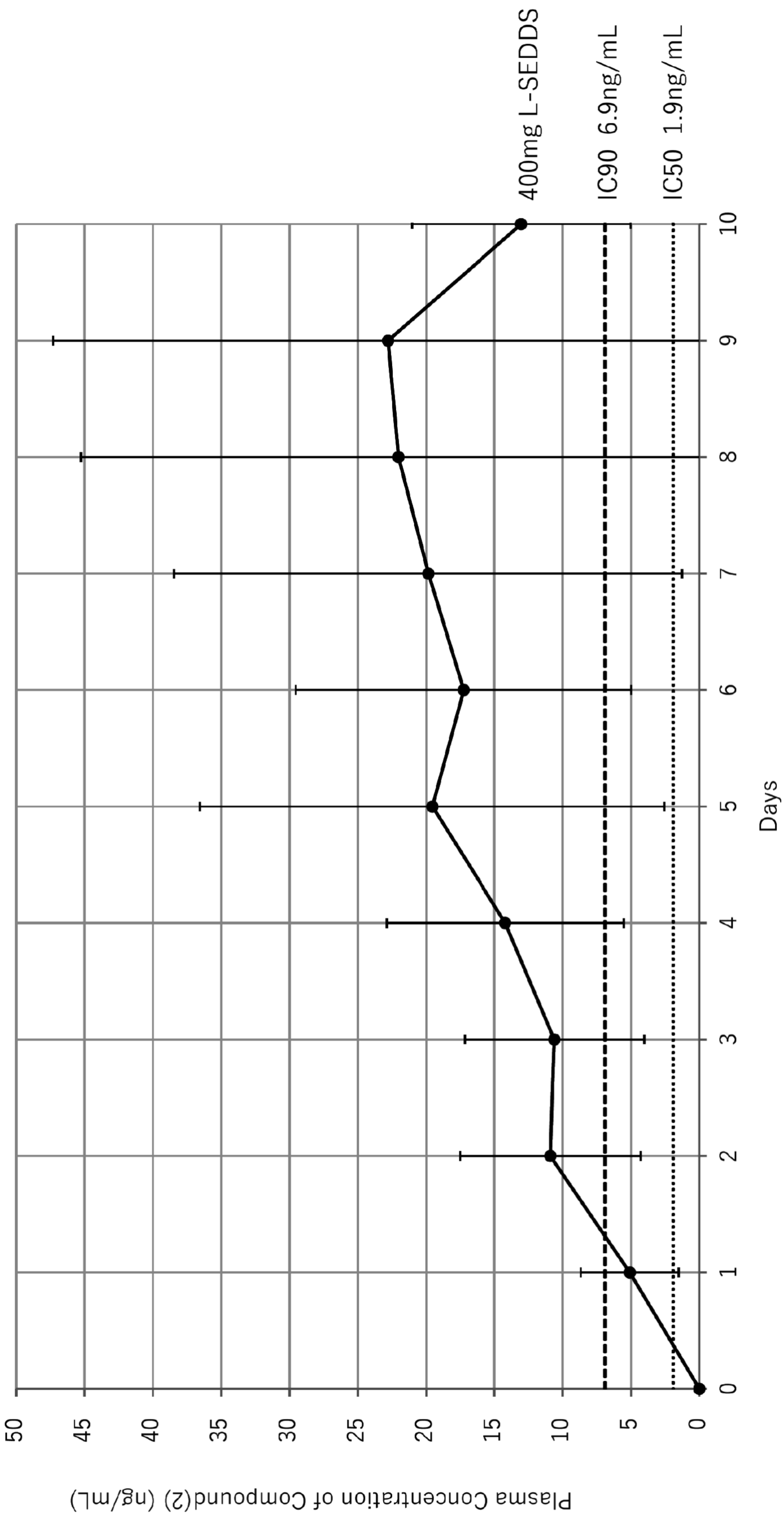
Plasma Concentration of Compound(2) (ng/mL)
0
5
10
15
20
25
30
35
40
45
50
Days
0
1
2
3
4
5
6
7
8
9
10
400mg L-SEDDS
IC90 6.9ng/mL
IC50 1.9ng/mL

FIG.2B

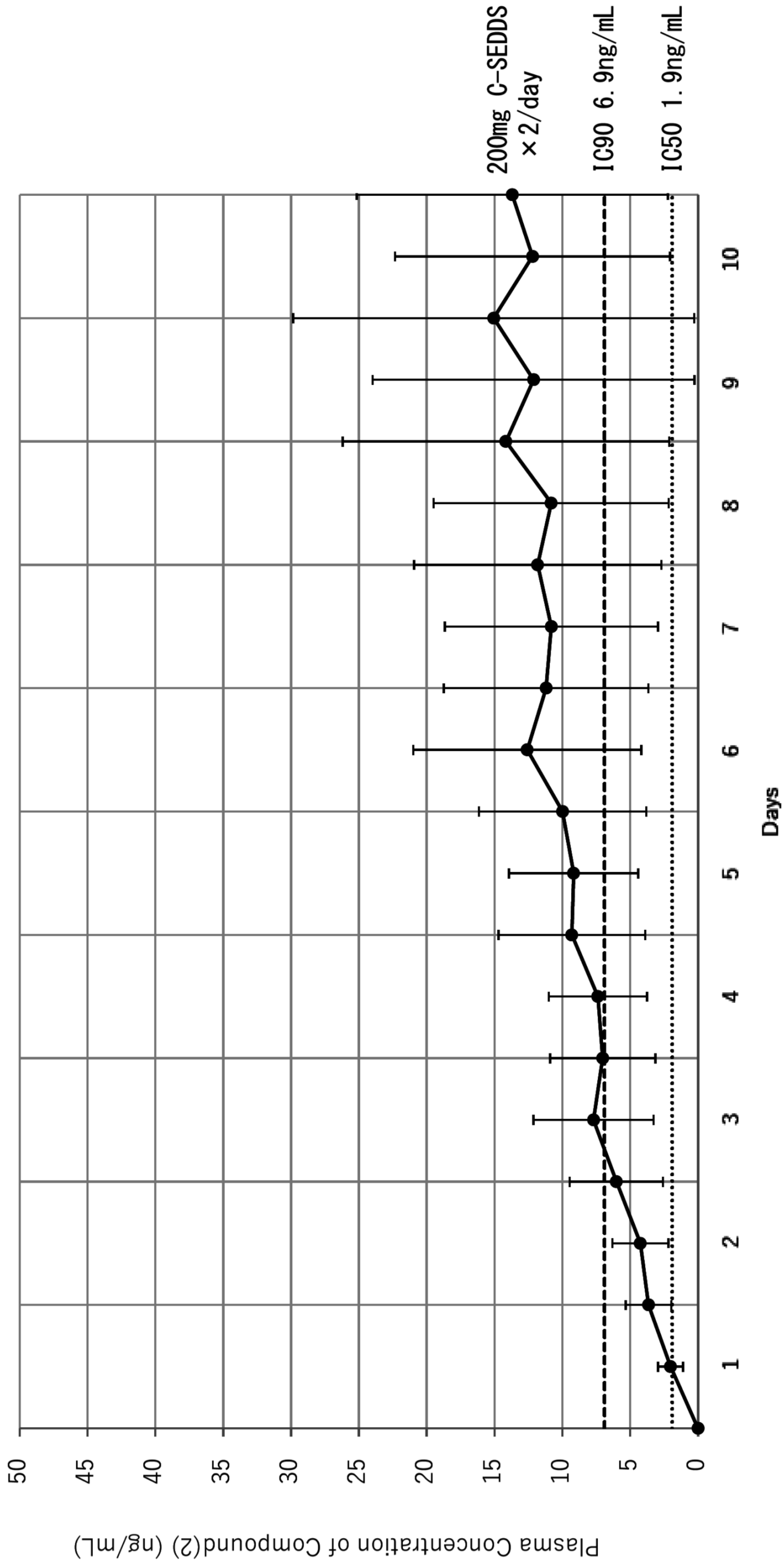
200mg C-SEDDS ×2/day
IC90 6.9ng/mL
IC50 1.9ng/mL
Plasma Concentration of Compound(2) (ng/mL)
0
5
10
15
20
25
30
35
40
45
50
Days
1
2
3
4
5
6
7
8
9
10

FIG.3A

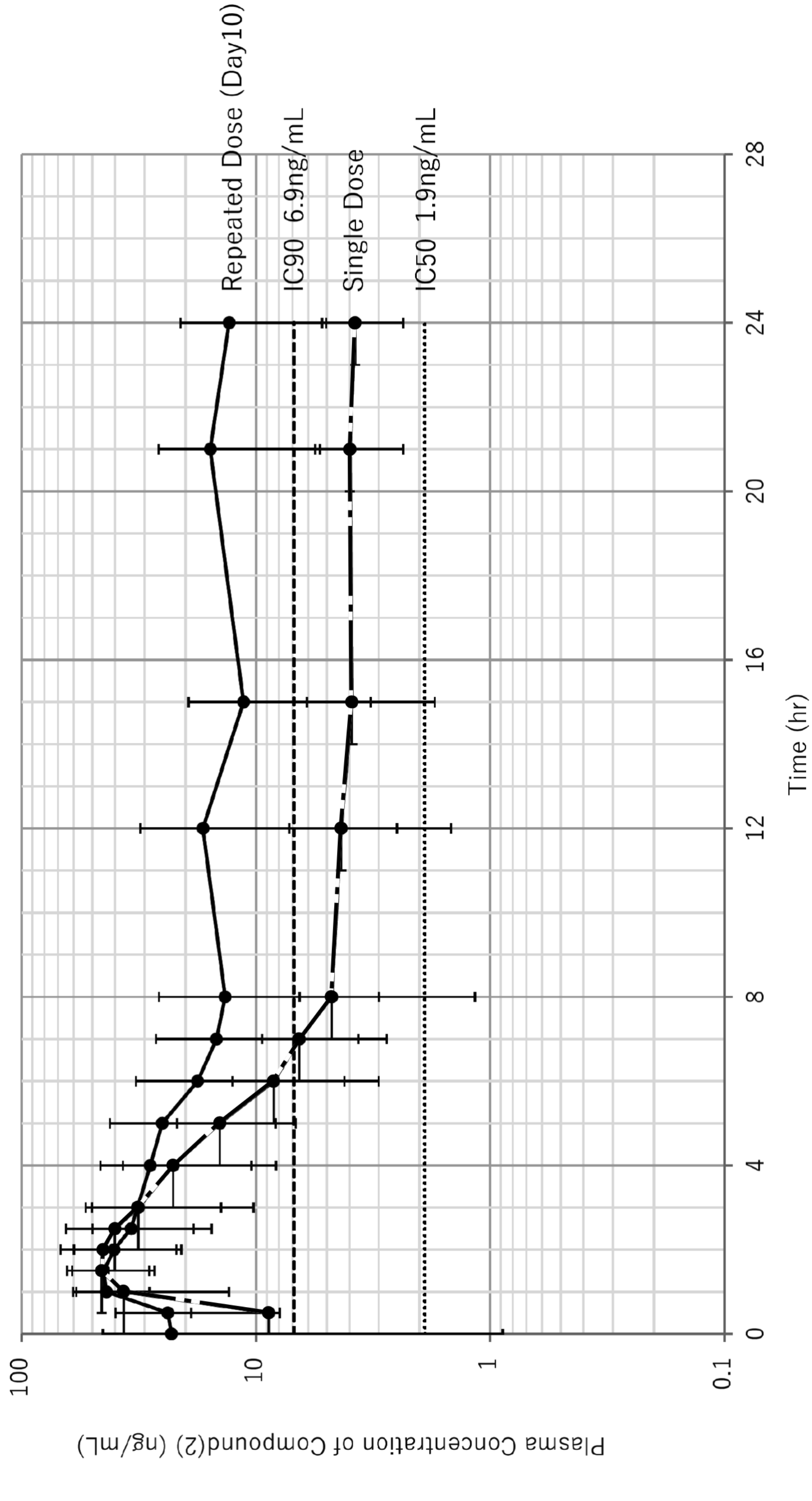
Repeated Dose (Day10)
IC90 6.9ng/mL
Single Dose
IC50 1.9ng/mL
0
4
8
12
16
20
24
28
Time (hr)
100
10
1
0.1
Plasma Concentration of Compound(2) (ng/mL)

FIG.3B

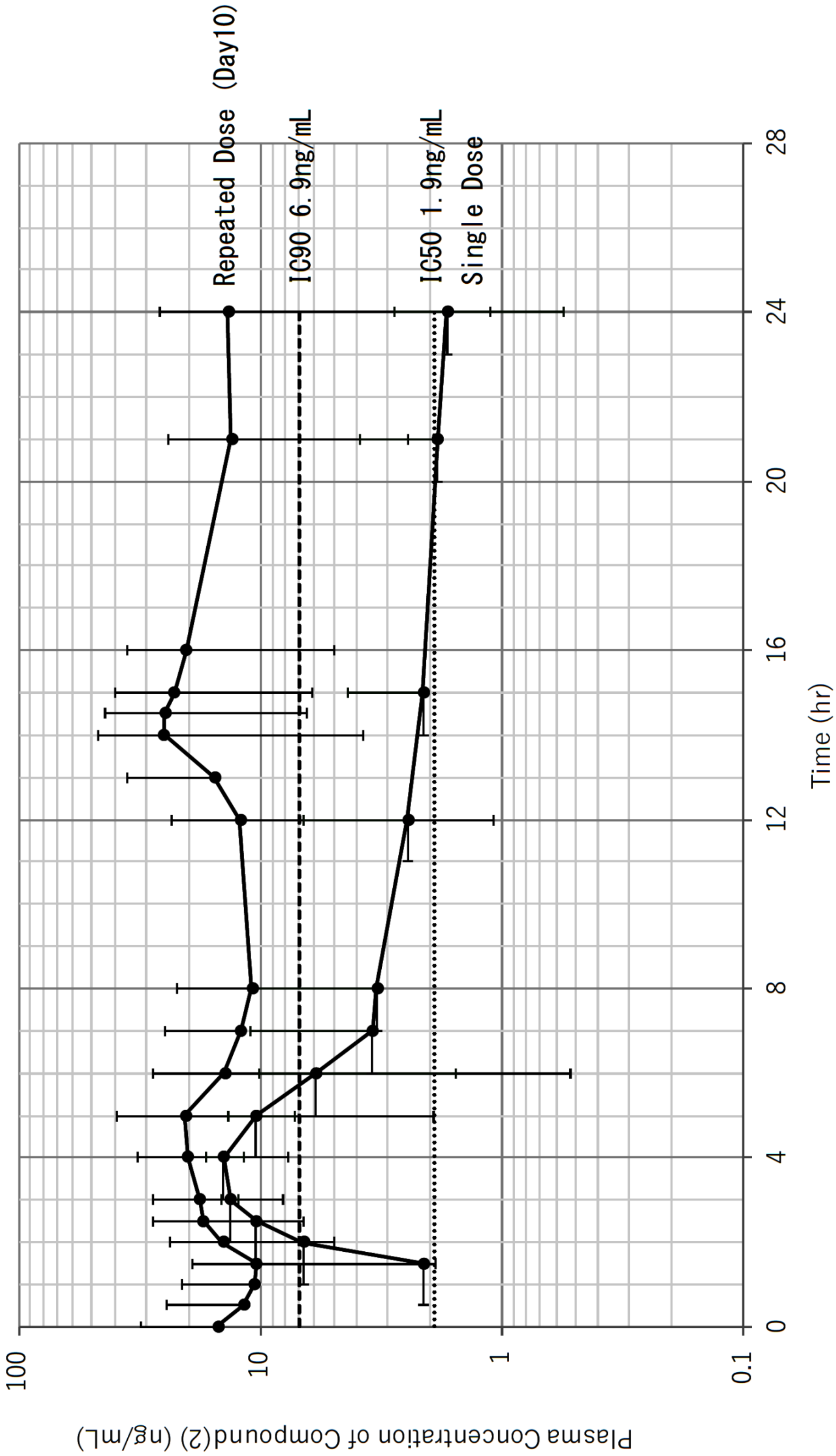

Repeated Dose (Day10)
IC90 6.9ng/mL
IC50 1.9ng/mL
Single Dose
Plasma Concentration of Compound(2) (ng/mL)
100
10
1
0.1
Time (hr)
0
4
8
12
16
20
24
28

FIG.4

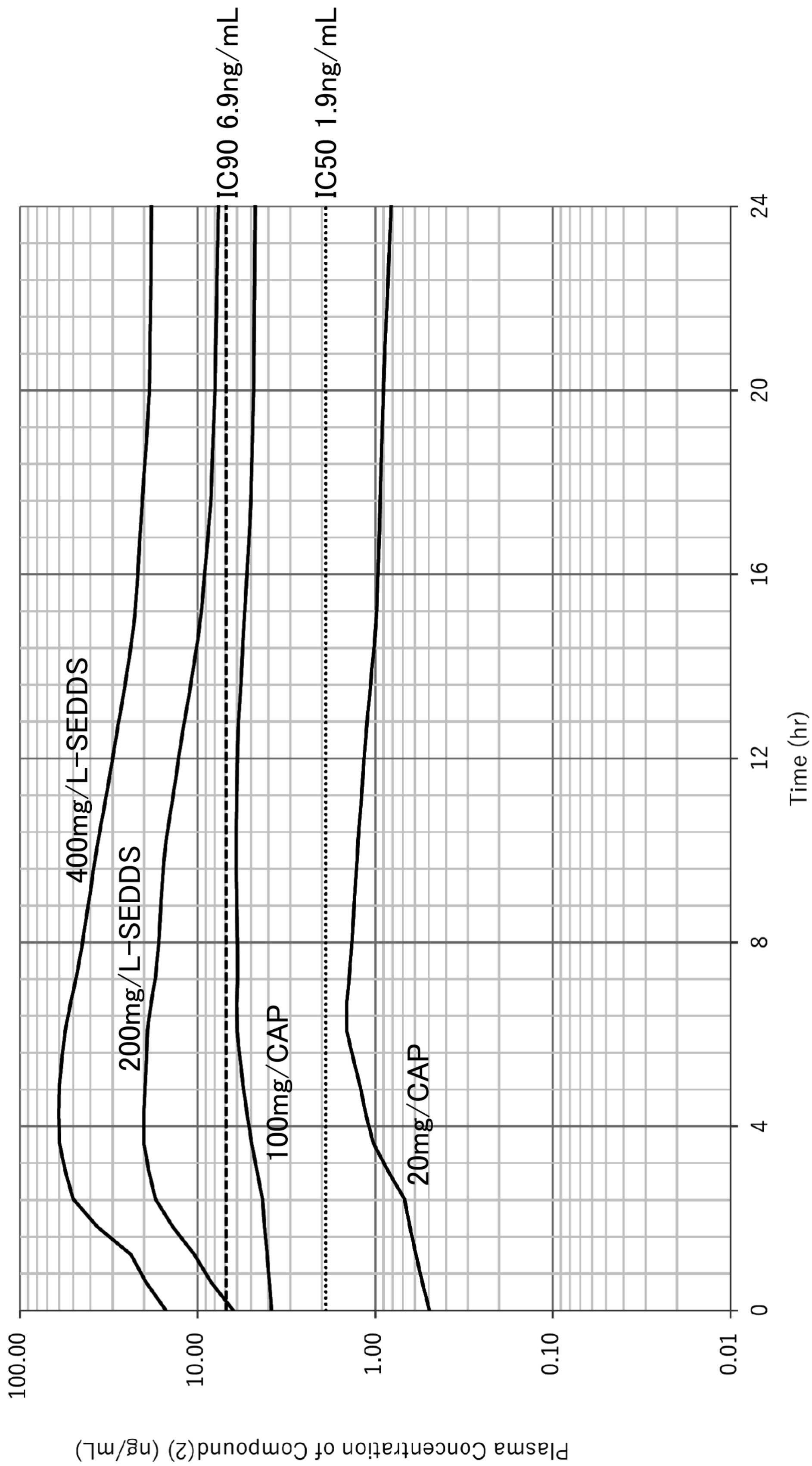

IC90 6.9ng/mL
IC50 1.9ng/mL
400mg/L-SEDDS
200mg/L-SEDDS
100mg/CAP
20mg/CAP
Time (hr)
0
4
8
12
16
20
24
Plasma Concentration of Compound(2) (ng/mL)
100.00
10.00
1.00
0.10
0.01

FIG.5

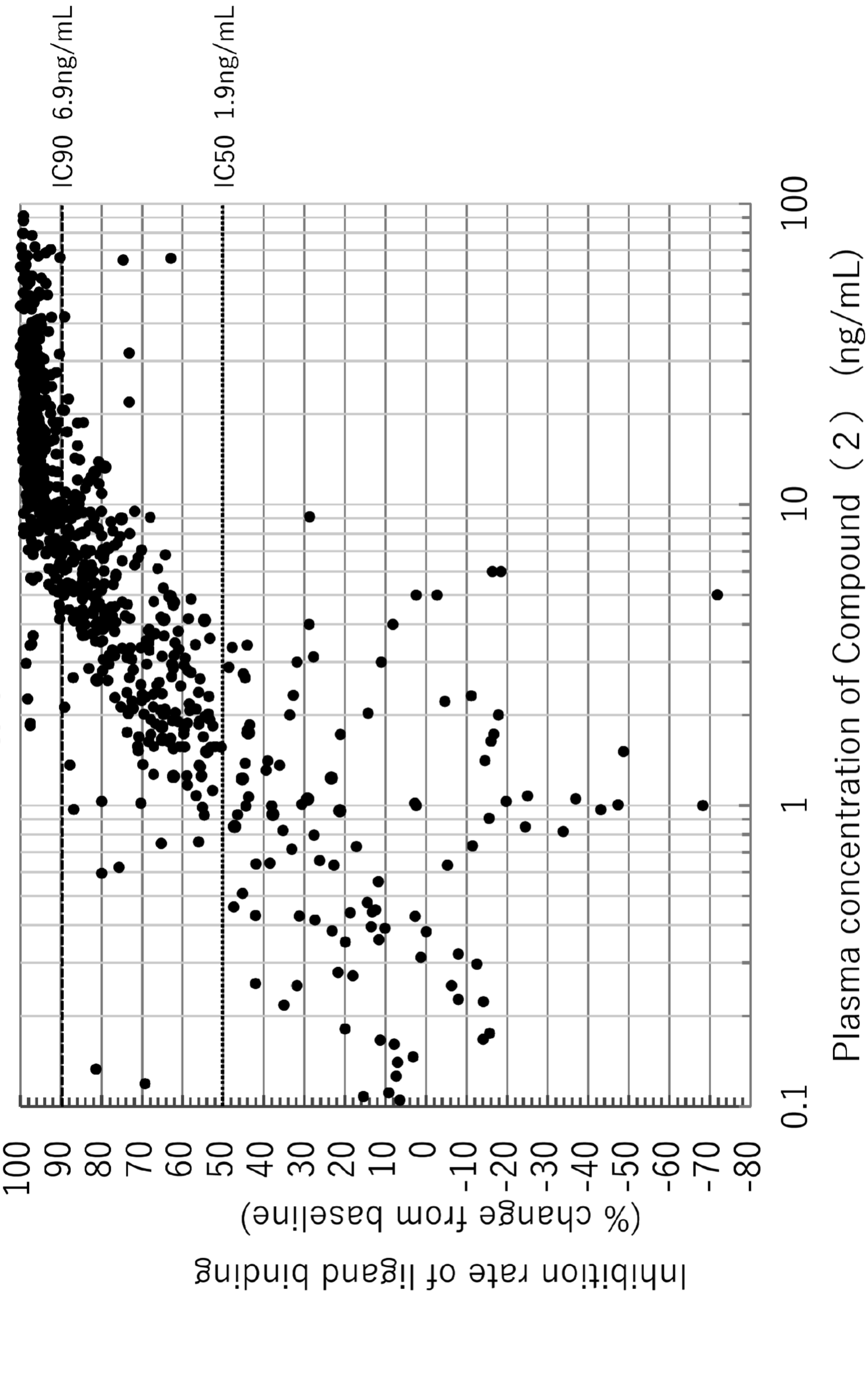
IC90 6.9ng/mL
IC50 1.9ng/mL
100
90
80
70
60
50
40
30
20
10
0
-10
-20
-30
-40
-50
-60
-70
-80
Inhibition rate of ligand binding
(% change from baseline)
0.1
1
10
100
Plasma concentration of Compound（2）(ng/mL)

FIG.6

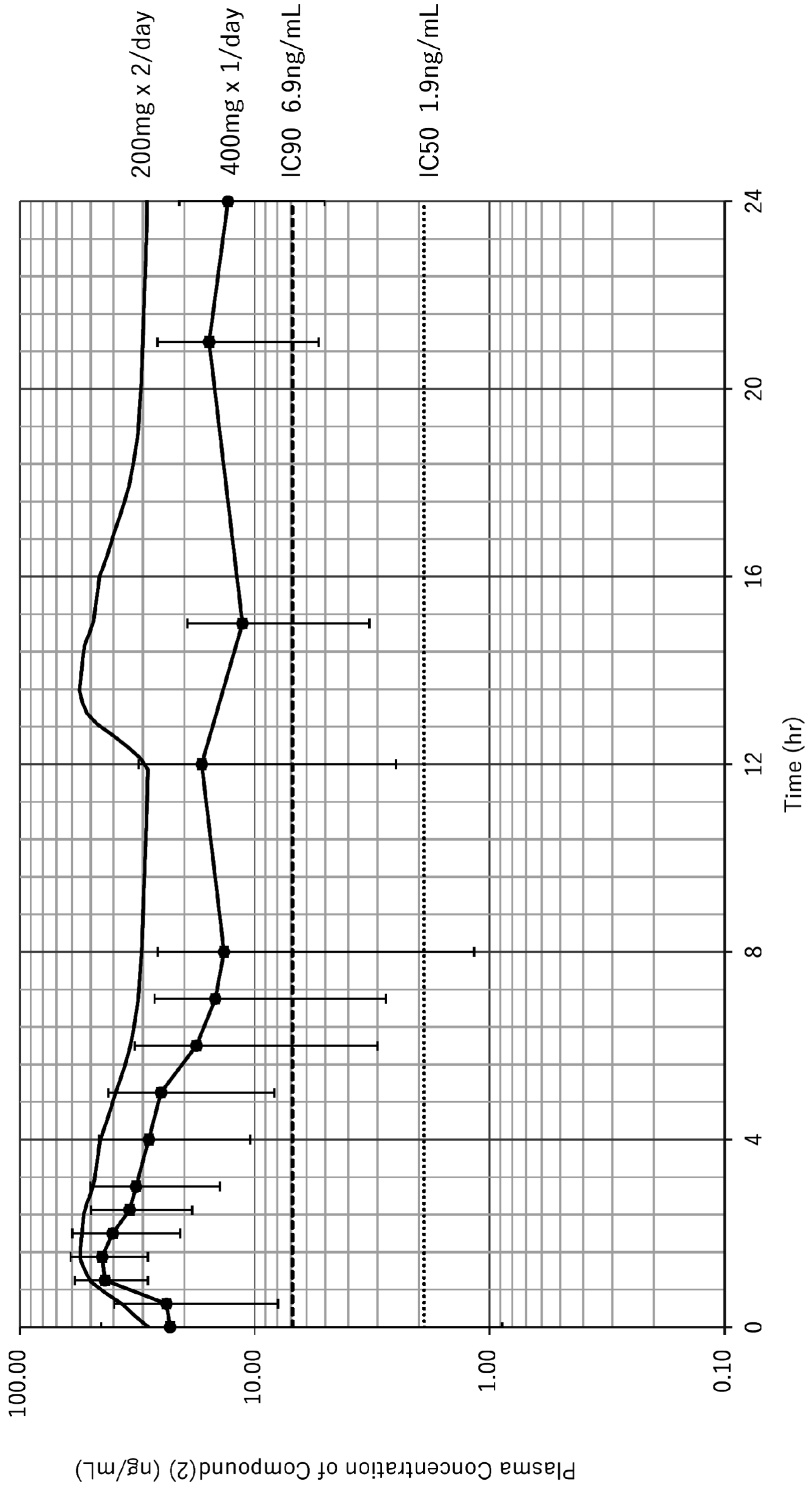
200mg x 2/day
400mg x 1/day
IC90 6.9ng/mL
IC50 1.9ng/mL
100.00
10.00
1.00
0.10
Plasma Concentration of Compound(2) (ng/mL)
0
4
8
12
16
20
24
Time (hr)

## INTERNATIONAL SEARCH REPORT

International application No.
**PCT/JP2024/014041**

**A. CLASSIFICATION OF SUBJECT MATTER**

***A61K 31/513***(2006.01)i; ***A61K 9/107***(2006.01)i; ***A61K 31/635***(2006.01)i; ***A61K 47/54***(2017.01)i; ***A61P 1/04***(2006.01)i; ***A61P 43/00***(2006.01)i

FI: A61K31/513; A61P1/04; A61K9/107; A61K47/54; A61P43/00 123; A61K31/635

According to International Patent Classification (IPC) or to both national classification and IPC

**B. FIELDS SEARCHED**

Minimum documentation searched (classification system followed by classification symbols)

A61K31/513; A61K9/107; A61K31/635; A61K47/54; A61P1/04; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2017/135472 A1 (EA PHARMA CO., LTD.) 10 August 2017 (2017-08-10) claim 23, paragraphs [0092], [0102] | 1-11 |
| A | 杉浦俊彦ほか, 経口α4 インテグリン阻害薬カロテグラスト メチル （カログラ錠１２０ｍｇ）の薬理学的特徴と臨床試験成績, 日薬理誌, 01 March 2023, 158(2):203-210, (SUGIURA, Toshihiko et al. Pharmacological and Clinical data of oral alpha 4 integrin antagonist, Carotegrast methyl, CAROGRA®. Folia Pharmacologica Japonica.) | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 June 2024** | **25 June 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2024/014041**

| Patent document cited in search report | Publication date (day/month/year) | Patent family member(s) | Publication date (day/month/year) |
|---|---|---|---|
| WO 2017/135472 A1 | 10 August 2017 | US 2019/400598 A1<br>paragraphs [0449], [0471]-[0473]<br>EP 3412660 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017135472 A


### Non-patent literature cited in the description


- **RIICHI HISAMATSU**. Ulcerative Colitis and Crohn's Disease Diagnostic Criteria and Treatment Guidelines. 2021